# EUROPEAN PATENT SPECIFICATION

(11) **EP 1 779 893 B1**
(45) Date of publication and mention of the grant of the patent: **15.06.2011**
(21) Application number: 05023337.8
(22) Date of filing: 26.10.2005
(51) Int. Cl.: A61Q 5/02, A61Q 5/06, A61Q 5/10, A61Q 5/12, A61K 8/34, A61K 8/73, A61K 8/81, A61K 8/33, A61K 8/89

(54) **Process for producing an aqueous composition**
Verfahren zur Vorbereitung einer wässrigen Zusammensetzung
Procédé de préparation d'une composition aqueuse

(43) Date of publication of application: 02.05.2007
(73) Proprietor: KPSS-Kao Professional Salon Services GmbH, 64297 Darmstadt (DE)
(72) Inventor: Hoffmann, Martin, 64673 Zwingenberg (DE)

(56) References cited:
- EP-A- 1 127 566
- EP-A- 1 224 927
- EP-A- 1 479 368
- EP-A- 1 504 748
- EP-A- 1 555 010
- EP-A- 1 604 640
- EP-A- 1 629 825
- WO-A-01/76543
- WO-A-2004/100908
- FR-A- 2 849 774
- US-A- 5 470 551
- US-A1- 2002 182 164
- US-A1- 2003 180 374
- US-A1- 2005 079 193
- US-B1- 6 280 712
- US-B1- 6 833 406
- "Ciba Salcare SC96 technical and processing data" 1998, , XP002374353 * the whole document *
- ""Ciba Salcare sc95 technical and processing data"" 1998, , XP002374354 * the whole document *
- "ultragel 300" 2003, , XP002374355 Retrieved from the Internet: URL:www.cosrheo.com/ug300.html> [retrieved on 2006-03-27] * the whole document *

## Description

The present invention relates to a process for producing aqueous cosmetic compositions comprising a water-soluble polymer in powder form.

Polymers are used for various purposes such as thickening, conditioning and gelling agents in various type of products. In cosmetic compositions and especially of hair care composition the polymers have found their wide range of application. On one hand, they are unavoidable conditioning agents, especially cationic ones, and on the other hand, they are consistency giving or enhancing agents which makes application and/or usage easy.

Majority of the polymers are supplied in powder form and they are conventionally mixed with the remaining part of the formulation after dissolving in major part of water available in water. Such a process is first of all timely, requires at least, when considering large scale production, two large vessels and, therefore, extremity costly as well. Furthermore, when preparing solution of powdery polymer in water usually heat is needed in order to shorten the preparation time by increasing dissolution rate and more importantly due to the aggregation of powder particles, lumpiness, the preparation time of such solution is extremely extended, which is not economical.

The present invention starts from the above problems and aims at effectively solving those.

The object of the present invention is that process for producing an aqueous cosmetic composition comprising a water-soluble powder polymer characterised in that powder polymers is first dispersed in a liquid medium selecled from di(ethylhexyl)carhonate, linear, cyclic or arylated silicones, and esters of aliphatic linear or branched carboxylic acids with a primary or secondary linear or branched alcohol, selected from isopropyl myristate, palmitate, stearate and isostearate, oleyl oleate, isocetyl stearate, hexyl laurate, dibutyl adipate, dioctyl adipate, myristyl myristate and oleyl erucate in which it is insoluble at a mixing weight ratio of powder polymer and liquid dispersion medium is in the range of 1:1 to 1:10 and subsequently combined with the remaining of the composition.

Polymer dispersions are available commercially under the trade name Salcare® comprising either mineral oil of a diacyl ester together with a surfactant. Nothing is disclosed on the use of the liquid mediums of the present invention for economically producing aqueous cosmetic composition comprising a water soluble polymer in powder form which is not soluble in the said liquid medium.

Furthermore it has also surprisingly found out that a composition comprising a powder polymers which forms a thixothropic gel in aqueous medium is produced easily without introducing air bubbles into the gel composition as bubbler are almost impossible to get rid off if not wished.

The following are definition within the meaning of the present invention.

With the term "water-soluble polymer" it is meant that at least at the concentration of usage in the composition, powder polymer forms a solution in water without leaving any particulate matter when inspected macroscopically by naked eye. Transparency of solution is not a criterion, as there are number of powder polymers which do not form transparent solutions when dissolved in water.

With the term "insoluble" it is meant that a particulate dispersion is obtained after mixing polymer in the liquid dispersion medium, it is preferred that consistency of mixture of powder polymer and liquid medium does not exceed or equal to 500 mPa.s measured at 20°C with an appropriate viscosimeter, preferably Brookfield viscometer using appropriate spindle and rotation speed and preferably is not increased from the viscosity of the dispersing liquid medium.

With the term "liquid" it is meant that the dispersion medium is liquid at approximatety 20°C and more preferably liquid at temperatures above 5°C,

Examples to powder polymers without making any limitation are those of natural polymers such as cellulose, guar gum, xanthan gum and their derivatives and of cationic polymers such as Polyquaternium 10, cationic cellulose commercially available under trade names Celquat, Polymer JR, Polyqauternium 37 which is commercially available in powder form under the trade name Ultragel, and anionic acrylate polymers and their derivatives such as available under the trade name Carbopol.

As a liquid dispersion medium for powder polymers, suitable ones are of lipophilic ones with a viscosity of less than or equal to 500 mPa.s, preferably in the range of 1 to 400mPa.s and more preferably in the range of 1 to 350 mPa.s and most preferably 1 to 250 mPa.s measured at 20°C with an appropriate viscosimeter, preferably a capillary viscosimeter.

For the selection of the dispersion liquid medium for powder polymers, an important criterion is that it should be compatible with the remaining of the composition and not effecting its stability as well as its preferred physical appearance.

It has as well been found out that the amount of the liquid dispersion medium used should be kept as low as possible. Therefore, it is preferred that the weight ratio of powder polymer to liquid dispersion medium is in the range of 1:1 to 1:10, more preferably 1:1 to 1:5 and most preferably 1:1 to 1:2.

It is further preferred form of the invention that the composition produced according to the process disclosed herewith, comprises powder polymer at a concentration of 0.01 to 10%, preferably 0.01 to 7.5%, more preferably 0-1 to 5% by weight, calculated to total composition.

Compositions produced according to the process of the present invention are aqueous cosmetic compositions. Examples to those without making any limitation are hair and body cleansing preparations, hair conditioners, hair colours, skin creams or lotions, hair permanent shaping compositions, scalp products, hair styling gel, etc.

In the case that the compositions produced according to the process of the present invention are cleansing preparations, and then, they comprise at least one foaming surfactant selected from anionic, non-ionic and amphoteric ones. Examples to those without making any limitation are anionic surfactants such as alkyl sulfates or alkyl ether sulfates and their salts such as sodium laureth sulphate, alkyl carboxylates or alkyl ether carboxylates or alkyl amido ether carboxylates and their salts.

sulfosuccinates, non-ionic ones such as alkyl glucosides according to general formula

R₁-O-(CH₂CH₂O)ₙ-Zₓ,

wherein R₁ is an alkyl group with 8 to 20, preferably 10 to 14 carbon atoms, Zₓ is a saccharide group with 5 to 6 carbon atoms, n stands for a number from 0 to 10, and x is a number between 1 and 5, preferably 1.1 to 2.5, sorbitan esters, amin oxides, amphoteric ones such as betaine types, alkyl betaines or alkyl amido betaines, and alkyl sulphobetaines.

Conditioning composition especially for hair comprises further conditioning ingredients such as cationic polymers known with the INCI name Polyquaternium or Quaternium, cationic surfactants, oily ingredients etc. Cationic surfactsnts are selected form the compounds of the general formula 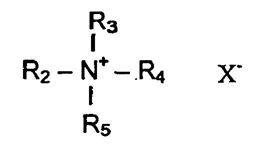 where R₂ is a saturated or unsaturated, branched or non-branched alkyl chain with 8-22 C atoms or

R₆ CO NH (CH₂)ₙ

where R₆ is saturated or unsaturated, branched or non-branched alkyl chain with 7-21 C atoms and n has value of 1 - 4, or

R₇ CO O (CH₂)ₙ

where R₇ is saturated or unsaturated, branched or non-branched alkyl chain with 7-21 C atoms and n has value of 1 - 4, and
R₃ is unsaturated or saturated, branched or non-branched alkyl chain with 1 to 22 C atoms or

R₆ CO NH (CH₂)ₙ

or

R₇ CO O (CH₂)ₙ

where R₆ , R₇ and n are same as above, and
R₄ and R₅ are lower alkyl or hydroxyalkyl or polyhydroxyalkyl with 1 to 4 C atoms and X is chloride, bromide, methosulfate.

Examples to cationic polymers Polyquaternium 6, Polyquaternium 7, Polyquaternium 10, Polyquaternium 11, Polyquaternium 16, Polyquaternium 22 and Polyquaternium 28. It has been found out that especially those or cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60. Quaternium-61, Quatemium-72, Quaternium-78, Quaternium-80, Quaternium-81, Quaternium-81, Quatemium-82, Quaternium-83 and Quaternium-84.

The cationic polymers also include the quaternized products of graft polymers from organopolysiloxanes and polyethyl oxazolines described in EP-A 524 612 and EP-A 640 643.

Examples to cationic surfactants are particularly cetyl trimethly ammonium chloride, steartrimonium chloride, behentrimonium chloride, stearamidopropyl trimonuim chloride, behenamidopropylethyldimonium ethosulfate, behenamidopropyltrimonium methosulfate, cocamidopropytrimonium chloride, cocotrimonim chloride, palmitamidopropyltrimonum chloride, distearyldimonium chloride, dipalmitoylethylhydroxyethylmonium chloride, dioleoylethyl dimethyl ammonium methosulfate, dioleoylethyl hydroxyethylmonium methosulfate, dilinolamidopropyldimonium chloride, dioleylethyl hydroxyethylmonium chloride, dipalmitoylethyldimonium chloride.

In the case that an emulsion composition is prepared so it contains at least one fatty alcohol. The fatty alcohols preferred are linear or branched, saturated or unsaturated with 10 to 24, preferably 12 to 22 carbon atoms in its alkyl chain. Nonlimiting examples are myristyl alcohol, cetyl alcohol, stearyl alcohol, palmityl alcohol, oleyl alcohol, lauryl alcohol, coconut alcohol, palm alcohol, behenyl alcohol, arachidyl alcohol and their mixtures. Preferred are myristyl alcohol, cetyl alcohol, stearyl alcohol, palmityl alcohol, oleyl alcohol, behenyl alcohol and their mixtures.

Emulsion compositions comprise in addition to fatty alcohols at least one emulsifier selected from non-ionic, anionic, cationic and amphoteric surfactants. In addition to the above disclosed surfactants, preferred are C₁₀-C₂₂-fatty alcohol ethoxylates. Especially suited C₁₀-C₂₂-fatty alcohol ethers are the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16", "Ceteareth-20".

Composition produced according to present invention can be a hair colouring composition and comprise at least one dyeing agent selected from oxidative dye precursors, eventually in combination with one or more coupling agent, direct acting dyestuffs. The direct dyes referred are cationic, anionic, neutral and of plant dyes.

Suitable cationic dyestuffs are in principal those available on the market for hair colouring applications. Some examples to those are: Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 51 Basic Red 76, Basic Violet 1, Basic Violet 2, Basic Violet 3, Basic Violet 10, Basic Violet 14 and Basic Yellow 57, Basic Orange 31 and Basic Yellow 87. For this purpose, special reference is made to the PCT application WO 95/15144 of Ciba-Geigy AG. All cationic dyes disclosed therein are included here by reference.

Anionic dyes may as well be used either alone or in combination with cationic direct dyes. The suitable ones are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Additionally, the coloring compositions may comprise neutral dyes (HC dyes), so called nitro dyes either alone or in addition to the cationic and/or anionic direct dyes. Concentration of those can typically be in the range of 0.001 to 2%, preferably 0.01 to 1.5% and more preferably 0.05 to 1% by weight calculated to total aqueous composition.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs may also be used in combination with cationic direct-acting dyestuffs, for example henna (red or black), alkanna root, laccaic acid, indigo, logwood powder, madder root and rhubarb powder, etc.

Oxidative dye precursors are pyrazole derivatives are such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 1-methyl-4,5-diaminopyrazole, 1-methylethyl-4,5-diaminopyrazole, 1-phenylmethyl-4,5-diaminopyrazole, 1-methyl-4,5-diaminopyrazole, 1-(4-methylphenyl)methyl-4,5-diaminopyrazole, 1-methyl-3-phenyl-4,5-diaminopyrazole and the water-soluble salts. Further suitable are tetraaminopyrimidines are in particular 2,4,5,6-tetraaminopyrimidine and the lower alkyl derivatives thereof; suitable triaminohydroxypyrimidines are, for example 4-hydroxy-2,5,6-triaminopyrimidine, 2-hydroxy-4,5,6-triaminopyrimidine and 5-hydroxy-2,4,6-triaminopyrimidine; suitable mono- and diamino dihydroxypyrimidines are, for example, 2,6-dihydroxy-4,5-diaminopyrimidine, 2,4-diamino-6-hydroxy-pyrimidine or 4,6-dihydroxy-2,5-diaminopyrimidine or the water-soluble salts thereof, aminophenol derivatives such as 4-aminophenol, 4-amino-3-methylphenol, 2-chloro-4-aminophenol, 2,6-dichloro-4-aminophenol, 2,4-diamino-phenol, 2,6-dibromo-4-aminophenol and/or 2-aminophenol and water-soluble salts thereof, furthermore, phenylenedimanine derivatives such as 2,5-diamino-toluene, 2-n-propyl or 2-ethyl-p-phenylene-diamine, 2,6-dimethyl-p-phenylenediamine, 2-(2,5-diaminophenyl) ethanol, 1-amino -4-bis-(2'-hydroxy-ethyl)aminobenzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethylamino-4-aminobenzene, 1-hydroxy-2,5-diamino-4-methyl benzene, 1-hydroxymethyl-2,5-diaminobenzene, 1.3-dimethyl-2,5-diaminobenzene, 1,4-diamino isopropyl benzene and/or 1-amino-4-β-hydroxypropyl aminobenzene or the water-soluble salts thereof.

Coupling substance, are resorcinol, 2-methyl resorcinol, 4-amino-2-methylphenol, 4-chlororesorcinol, 2-amino-4-chlorophenol, 5-amino-4-methoxy-2-methylphenol, 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxypyridine, 2-dimethyl-amino-5-aminopyridine, 2,6-diaminopyridine, 1,3-diaminobenzene, 1-amino-3-(2'-hydroxyethylamino)benzene, 1-amino-3-[bis(2'-hydroxyethyl) amino]benzene, α-naphthol, 4,6-dichlororesorcinol, 1,3-diamino-toluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 2,6-dihydroxyethylamino toluene, 5-amino-2-methoxyphenol and/or 1-methoxy-2-amino-4-(2'-hydroxyethyl amino)benzene or the water-soluble salts thereof.

Concentration of any hair dye should be in the range of 0.001 to 5% by weight, calculated to total composition, in the case of oxidative dyes excluding the oxidizing agent which may be hydrogen peroxide.

The compositions may contain organic solvents, for example, as penetration enhancer such as ethanol. propanol, isopropanol, benzyl alcohol, benzyloxyethanol, ethylene carbonate, propylene carbonate, phenoxyethanol, butanol, isobutanol, cyclohexane, cyclohexanol, hexyleneglycol, ethylene glycol, butylenes glycol, propylene glycol, benzyl glycol, ethylene glycol monoethylether, ethylene glycol monobutyl ether, ethylene glycol monophenyl ether, 1-phenylethylalcohol, 2-phenylethylalcohol, o-methoxyphenol. Concentration of organic solvents in the conditioner composition can be in the range from 0.1 to 50% by weight, calculated to total composition.

Compositions may further comprise UV filters for protection of hair or skin, in the case of a cosmetic composition, from environmental influences such as loss of elasticity, loss of hair colour (bleaching effect of sun light). The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4.'-methylbenzophenone, 3-benzylidenecampher, 3-(4'-sulfo)-benzyl-idenebornane-2-one and the salts thereof and/or 3-(4'-methyl benzylidene)-DL-campher.

The preferred amount of the UV-absorber ranges from about 0.01 % to 5%, by weight, calculated to the total composition.

The pH of the compositions, in the case of cosmetic compositions, varies from 2 to 11. In the case of hair or skin care compositions particularly 2 to 7 and more particularly 2.5 to 6.0 and for colouring composition, especially those mixed with an oxidizing agent prior to application, it may be 6 to 11 and particularly 6 to 10. For adjusting the pH of the said conditioner compositions, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be adjusted in a way that conditioner composition so obtained has a pH value between 2 to 7. Typically concentration for acids can be 0.01 - 5% by weight, preferably 0.01 - 4% by weight, more preferably 0.05 - 2.5% by weight calculated to the total composition. In the alkaline range ammonium hydroxide and/or monoethanolamine are used for adjusting pH.

The composition may contain in principal any benefit agent serving the purpose of the usage and products designed for hair and skin may comprise further moisturisers and natural ingredients showing hair conditioning benefits.

The moisturising agents are selected from panthenol, polyols, such as glycerol, polyethylene glycols with molecular weight 200 to 20,000.

Additional natural plant extracts can as well form part of the compositions produced according to present invention. Those are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of almond, aloe, pineapple, artichoke, arnica, avocado, valerian, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, coconut, cocoanut, mango, peach, lemon, cornflower, wheat, apricot, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapon®" products, "Herbasol^{R}", "Sedaplant^{R}" and "Hexaplant^{R}". Extracts and the preparation thereof are also described in "Hagers Handbuch der pharmazeutischen Praxis", 4^{th} Ed..

The following examples are to illustrate the invention.

### Example 1

All values are in % by weight

| | |
|---|---|
| Ultragel 300 | 1.80 |
| Di(ethylhexyl)carbonate | 3.60 |
| Cetearyl alcohol | 3.00 |
| Cetrimonium chloride | 1.00 |
| Stearamidopropyldimethylamine | 1.00 |
| Isopropylmyristate | 0.25 |
| Dimethicone | 0.50 |
| Panthenol | 0.50 |
| Glycerin | 2.00 |
| Polyquaternium-11 | 0.30 |
| Lactic acid | q.s to pH 4.0 |
| Fragrance | q.s. |
| Preservative | q.s. |
| Water | to 100 |

The conditioner composition as above was prepared by first combining all ingredients in a vessel other than fragrance, Ultragel 300 (polyquaternium 37), di(ethylhexyl)carbonate and lactic acid, for pH adjustment, and heating the batch up to 70°C for melting fatty alcohol and homogenizing the mixture for about 5 min at a speed of 2000 rpm and subsequently Ultragel 300 dispersed in di(ethylhexyl)carbonate was added to the vessel and stirred until polymer was dissolved. Subsequently the batch is cooled down to around 35°C and fragrance was added. Finally pH was adjusted with lactic acid. The conditioner thus obtained has a pH value of 4 and has a viscosity of approximately 30,000 mPa.s measured with Brookfield viscosimeter at 20°C with spindle 4 at 10 rpm. The conditioner so obtained is opaque. The above composition was stable at room temperature for a period of longer than 6 months, at 40°C and 50°C for 3 months. The tests were interrupted after the said period at 40 and 50°C.

The above composition is produced with a batch size of 1.5 t in relatively short time (2 hrs) with using only one large vessel. A batch without dispersion of Ultragel 300 in a liquid medium, Di(ethylhexyl)carbonate, required at least 2 large vessels and production time was considerably longer, 3.5 hrs.

### Example 2

All values are in % by weight.

| | |
|---|---|
| Sodium laureth sulphate | 8.0 |
| Coco glucoside | 3.0 |
| Cocamidopropyl betaine | 3.0 |
| Panthenol | 0.5 |
| Polyquaternium 10 | 0.5 |
| Di(ethylhexyl)carbonate | 0.5 |
| Sodium chloride | q.s to viscosity 3000 mPa.s (Höppler at 20°C) |
| Preservative, fragrance | q.s. |
| Citric acid | q.s pH 5.2 |
| Water | q.s. to 100 |

The shampoo composition with the above formula was produced by first combining the surfactants, panthenol, and fragrance and major part of water. The batch was mixed until homogeneity and Polyquaternium 10 dispersed in Di(ethylhexyl)carbonate was added to the batch and finally viscosity and pH were adjusted.

The above production process shortened the production time to half compared to conventional method wherein polyquaternium 10 is first dissolved in water.

## Claims

1. Process for producing an aqueous cosmetic composition comprising at least one water soluble powder polymer **characterised in that** powder polymer is first dispersed in a liquid medium selected from di(ethylhexyl)carbonate, linear, cyclic or arylated silicones and esters of aliphatic linear or branched carboxylic acids with a primary or secondary linear or branched alcohol selected from isopropyl myristate, palmitate, stearate and isostearate, oleyl oleate, isocetyl stearate, hexyl laurate, dibutyl adipate, dioctyl adipate, myristyl myristate and oleyl erucate in which it is insoluble at a mixing weight ratio of powder polymer and liquid dispersion medium is in the range of 1:1 to 1:10 and subsequently combined with remaining of the composition.

2. Process according to claim 1 **characterised in that** liquid medium wherein powder polymer is dispersed has a viscosity of less than or equal to 500 mPa.s measured with a capillary viscosimeter at 20°C.

3. Process according to any of the preceding claims **characterised in that** final composition comprises powder polymer at a concentration of 0.01 to 10% by weight, calculated to total composition.

4. Process according to claims 1 to 3 **characterised in that** the composition is a shampoo and comprises additionally at least one foaming surfactant selected from non-ionic, anionic, amphoteric ones.

5. Process according to claims 1 to 4 **characterised in that** the composition is an emulsion and comprises additionally at least one fatty alcohol.

6. Process according to claims 1 to 5 **characterised in that** the composition comprises additionally at least one conditioning compound selected from cationic polymers, cationic surfactants and oily ingredients.

7. Process according to claim 6 **characterised in that** cationic surfactants are selected from compounds of general formula 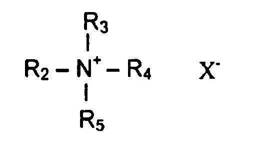 where R₂ is a saturated or unsaturated, branched or non-branched alkyl chain with 8-22 C atoms or
R₆ CO NH (CH₂)ₙ
where R₆ is saturated or unsaturated, branched or non-branched alkyl chain with 7-21 C atoms and n has value of 1 - 4,
or
R₇ CO O (CH₂)ₙ
where R₇ is saturated or unsaturated, branched or non-branched alkyl chain with 7-21 C atoms and n has value of 1 - 4, and
R₃ is unsaturated or saturated, branched or non-branched alkyl chain with 1 to 22 C atoms or
R₆ CO NH(CH₂)ₙ
or
R₇ CO O (CH₂)ₙ
where R₆, R₇ and n are same as above, and
R₄ and R₅ are lower alkyl or hydroxyalkyl or polyhydroxyalkyl with 1 to 4 C atoms and X is chloride, bromide, methosulfate.

8. Process according to claims 1 to 7 **characterised in that** the composition is a hair colouring composition comprising at least one dyestuff selected from oxidative dyestuffs precursors and direct dyes of anionic, cationic and non-ionic character.

## Patentansprüche

1. Verfahren zur Herstellung einer wässrigen kosmetischen Zusammensetzung die mindestens ein wasserlösliches Polymerpulver enthält, **dadurch gekennzeichnet, dass** das Polymerpulver zuerst in einem flüssigen Medium, ausgewählt aus Di(ethylhexyl)carbonat, linearen, cyklischen oder arylierten Silikonen und Estern von aliphatischen linearen oder verzweigten Carboxylsäuren mit einem primären oder sekundären linearen oder verzweigten Alkohol ausgewählt aus Isopropyl myristate, Palmitate, Stearate und Isostearate, Oleyl oleate, Isocetyl stearate, Hexyl laurate, Dibutyl adipate, Dioctyl adipate, Myristyl myristate und Oleyl erucate, in welchem es unlöslich ist, dispergiert wird, in einem Mischungs- Gewichtsverhältnis von Polymerpulver und flüssigem Dispersions-Medium im Bereich von 1 : 1 bis 1 : 10, und anschließend mit dem Rest der Zusammensetzung vereinigt wird.

2. Verfahren nach Anspruch 1, **dadurch gekennzeichnet, dass** das flüssige Medium, in dem das Polymerpulver dispergiert ist eine Viskosität von weniger oder gleich 500 mPa.s hat, gemessen mit einem Kapillar- Viskosimeter bei 20°C.

3. Verfahren nach einem der vorhergehenden Ansprüche, **dadurch gekennzeichnet, dass** die fertige Zusammensetzung Polymerpulver in einer Menge von 0,01 bis 10 Gew.- %, berechnet auf die Gesamtzusammensetzung, enthält.

4. Verfahren nach den Ansprüchen 1 bis 3, **dadurch gekennzeichnet, dass** die Zusammensetzung ein Shampoo ist und zusätzlich mindestens ein schäumendes Tensid, ausgewählt aus nicht ionischen, anionischen oder amphoteren Tensiden, enthält.

5. Verfahren nach den Ansprüchen 1 bis 4, **dadurch gekennzeichnet, dass** die Zusammensetzung eine Emulsion ist und zusätzlich mindestens einen Fettalkohol enthält.

6. Verfahren nach den Ansprüchen 1 bis 5, **dadurch gekennzeichnet, dass** die Zusammensetzung zusätzlich mindestens eine konditionierende Verbindung, ausgewählt aus kationischen Polymeren, kationischen Tensiden und öligen Bestandteilen, enthält.

7. Verfahren nach Anspruch 6, **dadurch gekennzeichnet, dass** die kationischen Tenside ausgewählt sind aus Verbindungen der allgemeinen Formel 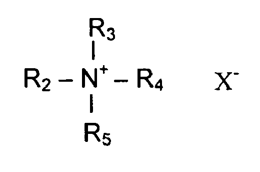 worin R₂ eine gesättigte oder ungesättigte, verzweigte oder nicht verzweigte Alkylkette mit 8 bis 22 C-Atomen ist, oder
R₆ CO NH (CH₂)ₙ
worin R₆ eine gesättigte oder ungesättigte, verzweigte oder nicht verzweigte Alkylkette mit 7 - 21 C-Atomen ist und n einen Wert von 1 - 4 hat, oder
R₇ CO O (CH₂)ₙ
worin R₇ eine gesättigte oder ungesättigte, verzweigte oder nicht verzweigte Alkylkette mit 7 - 21 C-Atomen ist und n einen Wert von 1 - 4 hat, und
R₃ eine ungesättigte oder gesättigte, verzweigte oder nicht verzweigte Alkylkette mit 1-22 C-Atomen ist, oder
R₆ CO NH(CH₂)ₙ
oder
R₇ CO O (CH₂)ₙ
worin R₆, R₇ und n gleiches wie oben sind, und
R₄ und R₅ niedrige Alkyl oder Hydroxyalkyl oder Polyhydroxyalkyl mit 1 - 4 C-Atomen sind und X Chlorid, Bromid, Methosulfat ist.

8. Verfahren nach den Ansprüchen 1 bis 7, **dadurch gekennzeichnet, dass** die Zusammensetzung ein Haarfärbemittel ist, das mindestens einen Farbstoff, ausgewählt aus oxidativen Farbstoffentwicklern und Direktfarbstoffen von anionischem, kationischem und nicht ionischem Charakter enthält.

## Revendications

1. Procédé de production d'une composition cosmétique aqueuse comprenant au moins un polymère en poudre soluble dans l'eau **caractérisé en ce que** le polymère en poudre est d'abord dispersé dans un milieu liquide choisi parmi le carbonate de di(éthylhexyle), des silicones linéaires, cycliques ou arylés et des esters d'acides carboxyliques aliphatiques linéaires ou ramifiés avec un alcool linéaire ou ramifié primaire ou secondaire choisi parmi le myristate, le palmitate, le stéarate et l'isostéarate d'isopropyle, l'oléate d'oléyle, le stéarate d'isocétyle, le laurate d'hexyle, l'adipate de dibutyle, l'adipate de dioctyle, le myristate de myristyle et l'érucate d'oléyle dans lequel il est insoluble à un rapport de mélange en poids de polymère en poudre et de milieu liquide de dispersion dans la plage de 1:1 à 1:10 et ensuite combiné avec le reste de la composition.

2. Procédé selon la revendication 1 **caractérisé en ce que** le milieu liquide dans lequel le polymère en poudre est dispersé a une viscosité inférieure ou égale à 500 mPa.s mesurée avec un viscosimètre capillaire à 20°C.

3. Procédé selon l'une quelconque des revendications précédentes **caractérisé en ce que** la composition finale comprend le polymère en poudre à une concentration de 0,01 à 10% en poids, calculée sur la composition totale.

4. Procédé selon les revendications 1 à 3 **caractérisé en ce que** la composition est un shampoing et comprend en outre au moins un agent tensioactif moussant choisi parmi des agents tensioactifs non ioniques, anioniques, amphotères.

5. Procédé selon les revendications 1 à 4 **caractérisé en ce que** la composition est une émulsion et comprend en outre au moins un alcool gras.

6. Procédé selon les revendications 1 à 5 **caractérisé en ce que** la composition comprend en outre au moins un composé de conditionnement choisi parmi des polymères cationiques, des agents tensioactifs cationiques et des ingrédients huileux.

7. Procédé selon la revendication 6 **caractérisé en ce que** les agents tensioactifs cationiques sont choisis parmi des composés de formule générale 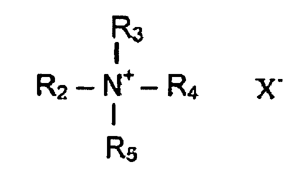 où R₂ est une chaîne alkyle saturée ou insaturée, ramifiée ou non ramifiée avec 8 à 22 atomes de C ou
R₆ CO NH (CH₂)ₙ
où R₆ est une chaîne alkyle saturée ou insaturée, ramifiée ou non ramifiée avec 7 à 21 atomes de C et n a une valeur de 1 à 4,
ou
R₇ CO O (CH₂)ₙ
où R₇ est une chaîne alkyle saturée ou insaturée, ramifiée ou non ramifiée avec 7 à 21 atomes de C et n a une valeur de 1 à 4, et
R₃ est une chaîne alkyle insaturée ou saturée, ramifiée ou non ramifiée avec 1 à 22 atomes de C ou
R₆ CO NH (CH₂)ₙ
ou
R₇ CO O (CH₂)ₙ
où R₆, R₇ et n sont les mêmes que ci-dessus, et
R₄ et R₅ sont un alkyle, ou un hydroxyalkyle ou un polyhydroxyalkyle inférieurs avec 1 à 4 atomes de C et X est un chlore, un brome, un méthosulfate.

8. Procédé selon les revendications 1 à 7 **caractérisé en ce que** la composition est une composition de coloration des cheveux comprenant au moins un colorant choisi parmi des précurseurs de colorants oxydatifs et des colorants directs de caractère anionique, cationique et non ionique.
